# EUROPEAN PATENT APPLICATION

(11) **EP 4 464 237 A1**
(43) Date of publication of application: **20.11.2024**
(21) Application number: 23173521.8
(22) Date of filing: 16.05.2023
(51) Int. Cl.: A61B 5/00, A61N 5/10, G16H 10/60, G16H 50/20, G16H 50/30, G06N 20/00

(54) **CONTROLLING RADIOTHERAPY TREATMENT**

(71) Applicant: Koninklijke Philips N.V., 5656 AG Eindhoven (NL)
(72) Inventor: HENDRIKS, Cornelis Petrus, Eindhoven (NL); BULUT, Murtaza, Eindhoven (NL); VAN EE, Raymond, 5656AG Eindhoven (NL); STEMKENS, Bjorn, Eindhoven (NL); COX, Lieke Gertruda Elisabeth, Eindhoven (NL); LAVEZZO, Valentina, Eindhoven (NL)
(74) Representative: Philips Intellectual Property & Standards

(57) **Abstract**

Proposed concepts aim to provide schemes, solutions, concepts, designs, methods, and systems pertaining to controlling radiotherapy treatment of a subject. Specifically, a digital twin (i.e. a set of virtual information constructs that mimics the structure, context, and behavior of the subject) is obtained that can output circadian rhythm information of the subject. For example, the digital twin may output circadian rhythm data of specific organs of the subject and/or a tumor of the subject. Such information may then be processed to identify a treatment parameter value(s), e.g. an optimal time window for treatment, a dosage regime, etc. Accordingly, the invention may provide information that can be used to improve the effectiveness of radiotherapy treatment, which accounts for the subject's specific circadian rhythm history.

## Description

### FIELD OF THE INVENTION

The present invention relates to the field of radiotherapy treatment, and more particularly to the field of controlling radiotherapy treatment of a subject.

### BACKGROUND OF THE INVENTION

It is well known that a subject's circadian rhythm has a significant effect on their physiological and mental state, as each of their organ's function according to a circadian rhythm. Distinct organs usually function synchronously with the subject's master circadian rhythm (i.e. body clock). If this is not the case, mental and physiological problems such as prostate and breast cancer may arise. Sometimes, due to jetlag, deep anesthesia, or irregular behavior and sleep, the subject's master circadian rhythm may become disrupted. Thus, understanding of a personal history of the irregularities in the circadian rhythm of the subject is important to fully understand an interaction between behavior and/or environment of the subject and their physiological state.

Furthermore, the circadian rhythm of a subject plays a key role in radiotherapy outcomes. The objective of radiotherapy in general is to maximize the TCP/NTCP ratio, where TCP stands for tumor control probability, and NTCP for normal tissue control probability. That is, radiotherapy aims to maximize tumor control and minimize toxicities for healthy tissues. Therapies to remove tumor cells are best administered when the tumor cells are dividing while at the same moment there is no cell division of healthy cells, thus maximizing the TCP to NTCP ratio. As every cell in the subject's body divides according to the circadian rhythm, it is clear that the circadian rhythm has a large role to play in ensuring effective radiotherapy treatment.

Moreover, digital twins have become increasingly of interest in the healthcare context. A digital twin of a subject is a set of virtual information constructs that mimics the structure, context and behavior of an individual, that is dynamically updated with data from its physical twin throughout its life-cycle. The digital twin typically receives data pertaining to the state of the subject, such as sensor readings or the like, based on which the digital twin can predict the actual or future status of the subject, e.g. through simulation. Thus, the digital twin enables the provision of information that would otherwise need to be acquired via time consuming and invasive testing and observations.

### SUMMARY OF THE INVENTION

The invention is defined by the claims.

According to examples in accordance with an aspect of the invention, there is provided a method for controlling radiotherapy treatment of a subject, the method comprising:
obtaining a digital twin adapted to output predicted circadian rhythms of the subject;
determining circadian rhythm data of the subject based on an output from the digital twin; and
processing the circadian rhythm data to identify a treatment parameter value for execution of radiotherapy treatment of the subject.

Accordingly, embodiments of the invention may ensure that the radiotherapy treatment of the subject is performed taking into account the circadian rhythm(s) of the subject. That is, because the circadian rhythm of the subject is responsible for the functions of various organs, as well as cell division rates of said organs and of a tumor of the subject, the circadian rhythm can greatly impact the effectiveness of radiotherapy treatment. Thus, by considering the circadian rhythm(s) of the subject, a treatment parameter (i.e. a time window for treatment, a dosage regime, etc.) may be appropriately identified that may result in an improved effectiveness of treatment.

Of course, circadian rhythms of the subject are difficult to measure and predict. The interaction between behavior of the subject (e.g., food intake, hydration, sleep), an environment of the subject (e.g., light exposure, ambient temperature), and the resulting circadian rhythm of the subject is complex and impractical to generalize. However, it has been realized that the use of a digital twin may provide an effective means for prediction of the circadian rhythm of the subject, even taking into account lifestyle and environmental changes and/or variations.

By way of brief explanation, the digital twin of a subject enables interactive visualization and physical insights of relevant health information of the subject. The digital twin comprises predictive models that provide projections of future health status and outcome of medical interventions. As a result, the digital twin provides a tool for effective prediction and assessment of the subject, accounting for a variety of factors to predict an internal state of the subject (e.g. circadian rhythms of the subject).

Thus, embodiments of the invention are based on the realization that the digital twin may be used for prediction of circadian rhythms of the subject, which in turn can be used to determine treatment parameters of effective radiotherapy treatment. In other words, a subject digital twin concept, which incorporates circadian rhythm information and time-stamped sensor data in a learning model, overcomes limitations with existing circadian rhythm prediction methods, forming an interface between the subject and a personalized history of circadian aspects. As a result, the invention provides a treatment parameter that is subject-specific/personalized, resulting in the potential for better outcomes, including better prognostics and predictions.

In some embodiments, the method may further comprise processing the circadian rhythm data to determine a target change in the circadian rhythm of the subject; and generating a recommendation based on the target change, the recommendation describing a change to an environment and/or a behavior of the subject.

At present, subjects are usually considered as passive elements of their radiotherapy treatment. That is, the subject may be assessed, and their therapy/treatment tailored accordingly. As a result, there are no means proposed to influence the subject's circadian rhythm. This appears to be a lost opportunity, with subjects often wanting to contribute to the success of their treatment, and there being many different opportunities within the treatment process by which a subject's circadian rhythm can be altered.

Therefore, some embodiments of the invention provide that the circadian rhythm data obtained from the digital twin be leveraged to determine a target change in the circadian rhythm. An ideal circadian rhythm may be determined/identified that may result in a greater chance of successful treatment, or more effective treatment in general. Once identified, recommendations may be produced that (if followed) aim to modify the subject's circadian rhythm to lead to a (greater chance of) more effective treatment.

For example, if it is determined that the main circadian rhythm of the subject (i.e. a body clock) needs to be shifted by a few hours, the recommendation may state that the subject should alter their sleep schedule by a few hours. Alternatively or additionally, the recommendation may be to change the lighting regime within a hospital environment so as to influence the subject's circadian rhythm.

Thus, this embodiment of the invention goes one step further from the realization that the circadian rhythm is greatly influential on the effectiveness of treatment. The above described embodiment provides that the circadian rhythm itself may be influenced (alongside treatment parameters) in order to enable more effective treatment.

In some cases, the target change in the circadian rhythm may be adapted to increase a phase difference between a circadian rhythm of healthy tissue of the subject and a circadian rhythm of a tumor of the subject.

By way of explanation, the cell division rate of the normal tissue follows the peripheral clock of the associated organ (i.e. follows the circadian rhythm of the subject). An effective time for radiotherapy administration is when the difference in cell division rate between the tumor and the normal tissue is at a maximum. Thus, by increasing the phase difference between the circadian rhythm of healthy tissue and tumor, there is an increase in the maximum difference between cell division rates of the tumor and normal tissue.

Put another way, this provides that the target change is such that the circadian rhythm of the subject is changed in an attempt to increase a maximum difference between the cell division rates of the tumor and the healthy tissue. As a result, the effectiveness of radiotherapy treatment may be improved.

Furthermore, in some embodiments of the invention, the recommendation may comprise at least one of a lighting regime, a temperature level regime, a nutrition regime, a hydration regime, a sleep regime, an exercise regime, and a medication regime.

These aspects may be considered in order to change the circadian rhythm of the subject in an effective manner.

In further embodiments, the circadian rhythm data of the subject may comprise circadian rhythm data of healthy tissue of the subject and circadian rhythm data of a tumor of the subject.

Embodiments of the invention may provide that processing the circadian rhythm data comprises comparing the circadian rhythm data of the healthy tissue of the subject and of the tumor of the subject. In this case, identifying the treatment parameter value may be based on a result of the comparison.

By considering both circadian rhythms, a treatment parameter value may be appropriately chosen. Indeed, a difference in the circadian rhythms is related to the ratio of tumor control probability (TCP) and normal tissue control probability (NTCP). Maximizing this ratio at the time that the radiotherapy treatment is performed is key for increasing the effectiveness of radiotherapy treatment.

Additionally, identifying the treatment parameter value may comprise determining a time window during which the phase difference between the circadian rhythm of healthy tissue of the subject and the circadian rhythm of a tumor of the subject meets a predetermined requirement. Thus, the treatment parameter value may be based on the identified time window.

As above, identifying a period of time (for performing radiotherapy treatment) in which the cell division rate of the tumor is high, while the cell division rate of the surrounding healthy tissue is low, enables effective radiotherapy treatment.

In some embodiments, the method may further comprise obtaining historical treatment data describing previous radiotherapy treatment of the subject; and adapting the identified treatment parameter value based on the treatment data. The historical treatment data may comprise at least one of a treatment parameter value, a tumor response, a complication rate, and an immune system response.

Accounting for previous treatments enables an improved selection of a treatment parameter value.

Furthermore, the treatment parameter value may comprise one of a treatment execution window, and a dosage delivery regime.

In some embodiments, the method may further comprise training the digital twin adapted to represent circadian rhythms of the subject using time-stamped circadian rhythm data of a tumor, time-stamped circadian rhythm data of healthy tissue, time-stamped circadian rhythm data of the body, and physiological data of the subject.

By training the digital twin using the time-stamped circadian rhythm data, and physiological data, an accurate prediction of the circadian rhythm of the subject may be obtained. In turn, an appropriate treatment parameter value can be identified.

In some embodiments, the physiological data may comprise lifestyle data, environmental data, and therapeutic data of the subject.

According to another example in accordance with an aspect of the invention, there is provided a computer program product comprising computer program code means which, when executed on a computing device having a processing system, cause the processing system to perform all of the steps of a method for controlling radiotherapy treatment of a subject.

According to additional examples in accordance with an aspect of the invention, there is provided a system for controlling radiotherapy treatment of a subject, the system comprising:
an interface configured to obtain a digital twin adapted to output predicted circadian rhythms of the subject;
a control unit configured to determine circadian rhythm data of the subject based on an output from the digital twin, and process the circadian rhythm data to identify a treatment parameter value for the execution of radiotherapy treatment of the subject.

In some embodiments, the system may further comprise a circadian adaptation unit configured to process the circadian rhythm data to determine a target change in the circadian rhythm of the subject, and generate a recommendation based on the target change, the recommendation describing a change to an environment and/or a behavior of the subject.
These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a block diagram representing a machine learning based digital twin;
Figs 2A and 2B shows graphs representing the interaction between circadian rhythms of the subject, and cell division rates over time;
Fig. 3 shows a set of curves representing normalized circadian rhythm signals of the master clock, normal tissue, and tumor of a subject;
Fig. 4 shows curves representing normalized circadian rhythm signals of the master clock and normal tissue, and an ultradian rhythm of a tumor;
Fig. 5 presents a flow diagram of a method for controlling radiotherapy treatment of a subject according to an aspect of the invention;
Fig. 6 presents a flow diagram of a part of a method for controlling radiotherapy treatment of a subject;
Fig. 7 presents a simplified block diagram of a system for controlling radiotherapy treatment of a subject according to another embodiment; and
Fig. 8 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to controlling radiotherapy treatment of a subject. Specifically, a digital twin (i.e. a set of virtual information constructs that mimics the structure, context, and behavior of the subject) is obtained that can output circadian rhythm information of the subject. For example, the digital twin may output circadian rhythm data of specific organs of the subject and/or a tumor of the subject. Such information may then be processed to identify a treatment parameter value(s), e.g. an optimal time window for treatment, a dosage regime, etc. Accordingly, the invention may provide information that can be used to improve the effectiveness of radiotherapy treatment, which accounts for the subject's specific circadian rhythm history.

Put another way, the proposed invention leverages circadian rhythm information in order to control radiotherapy treatment more effectively. Internal rhythms of the subject (including the circadian or ultradian rhythm of tumor matter, and circadian rhythm of healthy tissue surrounding the tumor) have a large impact on the effectiveness of the treatment. For example, when the rhythm of the tumor is at a peak, the cell division rate may be at a maximum, and therefore may be more susceptible to radiotherapy. The same may be true for healthy tissue, however susceptibility to radiotherapy for such tissue is undesirable in order to avoid problems (e.g. the generation of other tumorous cells).

Such circadian rhythm data can be difficult to obtain directly from the subject at all stages of their treatment. Therefore, the digital twin provides a means for generating such data in a reliable, accurate and unintrusive way. Indeed, the digital twin accounts for the various complex and interrelated workings of the body of the subject, as well as the interaction of such mechanisms with changes to the lifestyle and environment of the subject. Thus, the digital twin can also be used to generate/predict future circadian rhythm data taking into account hypothetical changes to the subject (e.g. treatment, lifestyle changes and changes to the environment).

To be clear, a subject's circadian rhythm has a significant effect on their physiological and mental state because each organ functions according to a circadian 24-hour rhythm. In addition, all cells in the body divide according to this rhythm. It is important that distinct organs function synchronously with the master circadian clock. If this is not the case mental and physiological problems such as prostate and breast cancer may arise. Sometimes, due to jetlag, deep anesthesia, and irregular behavior and sleep, the master clock becomes disrupted. Thus, personalization and personal history of the irregularities in the circadian rhythm of a subject are important for a full understanding of the circadian rhythm of the subject. This understanding is essential when radiotherapy or surgery is planned.

Therapies to remove tumor cells are best administered when the tumor cells are dividing while at the same moment there is no cell division of healthy cells. This is because being exposed to radiation, e.g. ultraviolet sunlight, X-ray from medical imaging or radiotherapy, can create tumor cells. This principle is also known as chronotherapy. Indeed, the objective of radiotherapy in general is to maximize the ratio between tumor control probability (TCP), and normal tissue control probability (NTCP). Thus radiotherapy aims to maximize tumor control and minimize toxicities provided to healthy tissues.

For radiotherapy, it is proposed to account for the circadian rhythms of normal (healthy) tissue and tumor tissue. Tumor cells, especially those from fast growing or less differentiated tumors, can also follow ultradian rhythms (having a period of a few hours). This provides an opportunity to find optimal time windows when radiosensitivity is high in tumor tissue while low in normal (healthy) tissues. Furthermore, radiotherapy may alter the tumor rhythm to become circadian, which must also be considered when optimizing a radiotherapy schedule on an individual basis.

As described above, it is recognized that circadian rhythm plays a significant role in radiotherapy outcomes (tumor control, side effects). Thus, embodiments of the invention utilize circadian rhythm information/data to enhance diagnosis and therapy.

Primarily, it has been realized that a digital twin concept, which incorporates circadian rhythm information and time-stamped sensor data in a learning model, overcomes limitations with existing radiotherapy control methods. That is, the digital twin can be used as predictive model for circadian rhythms of a subject. The digital twin forms the interface between the subject and educated decisions that relate to personalized history of circadian aspects. The benefit is a personalized and adaptive therapy approach, further than is currently possible, with potentially better outcomes, including better prognostics and predictions.

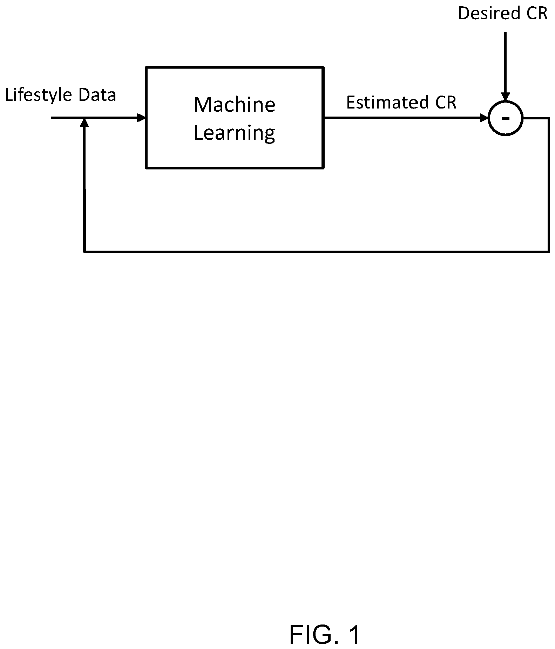

Furthermore, in some embodiments of the invention, the fact that subjects often want to contribute to their treatment is leveraged. This can help to improve the effectiveness of their treatment. Thus, with knowledge of circadian rhythms, coaching can be achieved to change the circadian rhythm. For example, a mobile phone application can be used to supply guidance/recommendations to alter the circadian rhythm. For example, the recommendation may be to drink less coffee, go to bed earlier or later, go for a walk, catch sunlight in the morning etc.
By way of explanation, Fig. 1 presents representing a machine learning based digital twin. In essence, a digital twin with circadian rhythm information can be trained and applied to achieve a desired circadian rhythm of the body (master clock) and the coupled organs (peripheral clocks), based on adapting the lifestyle of the patient (sleep, light, food, exercise etc.).

The digital twin of a subject offers advanced interactive visualization and physical insights of relevant health information of the subject. Combining visualization with predictive models that provide projections of future health status and outcome of medical interventions results in personalized clinical decision support. In turn, this enables improved diagnostics, treatment selection, and intervention selection in a healthcare process.

In other words, the digital twin accounts for the complex interactions between many aspects of a subject (e.g. lifestyle, received healthcare, surrounding environment). The digital twin of a subject (i.e. subject-specific computational simulations) may serve a number of purposes. Firstly, the digital twin (rather than the subject) may be subjected to a number of virtual tests, e.g. lifestyle regimes, treatment plans, etc., to determine which a change is most likely to result in a successful change in circadian rhythm of the subject. This reduces the number of tests that physically need to be performed on the actual subject. Secondly, the digital twin of a subject may be used to predict the onset, treatment (outcome) or development of medical conditions of the subject, as well as the current, past and future circadian rhythms of the subject.

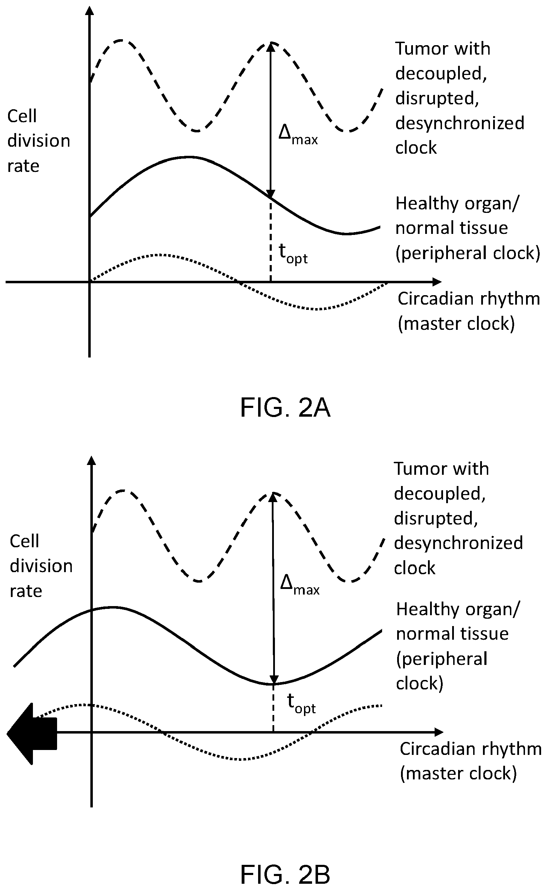

Turning to the concept of interaction between circadian rhythms, cell division rate, and treatment effectiveness, simplified graphs are presented in Figs 2A and 2B depicting cell division rates/circadian rhythms with time for different parts of a subject.

Specifically, Fig. 2A shows that the peripheral clock (i.e. the circadian rhythm of healthy organs/normal tissue) lags slightly behind the master clock (circadian rhythm of the subject). The circadian rhythm (or ultradian rhythm) of the tumor may be desynchronized from the master clock and/or the peripheral clock. Therefore, the cell division rates of normal tissue and of the tumor are desynchronized. This means that a difference between the cell division rates of said tumor and healthy tissue will vary over time. Identifying time-windows in which the cell division rate is above a predetermined threshold will enable more effective treatment.

In other words, the digital twin of the subject may be leveraged to determine the optimal time window for the radiotherapy administration. That is, when the cell division rate of the tumor is relatively high compared to the cell division rate of the normal tissue (shown by the arrow of Fig. 2A). The cell division rate of the normal tissue follows the peripheral clock of the specific organs, which follow with a delay the master clock. It is worth noting that the quantitative values of the cell division rates are not important, it is the difference (Δₘₐₓ) between the cell rates that matters. Δₘₐₓ relates to TCP/NTCP, and therefore an ideal time for radiotherapy administration, t = tₒₚₜ, is when the difference in cell division rate between the tumor and the normal tissue is maximum, Δ = Δₘₐₓ. It may well be the case (in fact it is likely) that the best time for treatment varies on a day-to-day basis (as a result of the difference in CR or due to the effect of radiotherapy).

Depending on the time delay between the master and the peripheral clock, and difference with the tumor clock, the optimal moment may also be a time interval. For example, when Δₘₐₓ stays substantially constant for a period.

The digital twin of the subject can capture the relations between the different complex parameters, i.e. between the tumor, its micro- and macro-environment, and the patient lifestyle are complex. Thus, the digital twin enables the accurate calculation of Δ, and therefore a best time to perform radiotherapy treatment.

Fig. 2B shows how, with a shift in the circadian rhythm of the subject (and therefore the rhythm of the healthy organ/normal tissue), Δₘₐₓ can be increased. Accordingly, the TCP/NTCP ratio may also be increased, leading to a more effective radiotherapy treatment if performed at t = tₒₚₜ.

Indeed, this may be achieved via lifestyle intervention, environmental change and/or treatment/therapy change. Recommendations for approaching a situation of circadian rhythm shift where this may be caused may be generated, such as a recommendation for an optimal lifestyle and daily schedules for therapy administration.

There is typically sufficient time to adapt the circadian rhythm of the subject before the start of radiotherapy. After it is decided that a subject requires radiotherapy, it often takes at least 10 days before the radiotherapy actually starts. Simply using light a misaligned biological clock can be shifted by approximately one hour a day, and with medication this may be increased. Furthermore, hospital rooms provide a perfect medium for an adjustment of the circadian rhythm. By adapting and closely controlling the light, nutrition, and sleep disruption (by a clinician), modifications of the circadian rhythm may be possible.

To paraphrase the above, in a conventional radiotherapy plan, the variation in cell division rates of the tumor and the normal tissue is not taken into account. Thus, the difference Δ at the time of therapy administration is unknown. In this case, the difference varies between -1 < Δ < 1 for normalized signals, i.e. divided by the amplitude minus the average value. If Δ is maximize, adjustment of the dose delivery to enhance TCP/NTCP in different known radiotherapy concepts is enabled. For example:
(i) Sparing. The total dose to the tumor is decreased. The rationale is that a same TCP can be achieved because the tumor receives the treatment while being more sensitive to radiation than average. Accordingly, the total dose to the healthy tissue is even lower than in a normal sparing approach, leading to the healthy tissue being exposed to fewer toxicities, and thus fewer related health issues.
(ii) Boosting. The total dose to the tumor is increased to achieve a higher TCP, while maintaining the same NTCP in the healthy tissue. Accordingly, the tumor is more radiosensitive, and the dose is higher. The healthy tissue also receives a higher dose, but this is counterbalanced by a lower radiosensitivity.
(iii) Fractionation. A personalized schedule may be designed that results in a maximum Δ during treatment, within a certain time constraint. For example, the total dose may be delivered over two weeks when Δ is at a maximum.
(iv) Dose painting. This is when Δ is maximizes for different regions in a heterogeneous tumor.

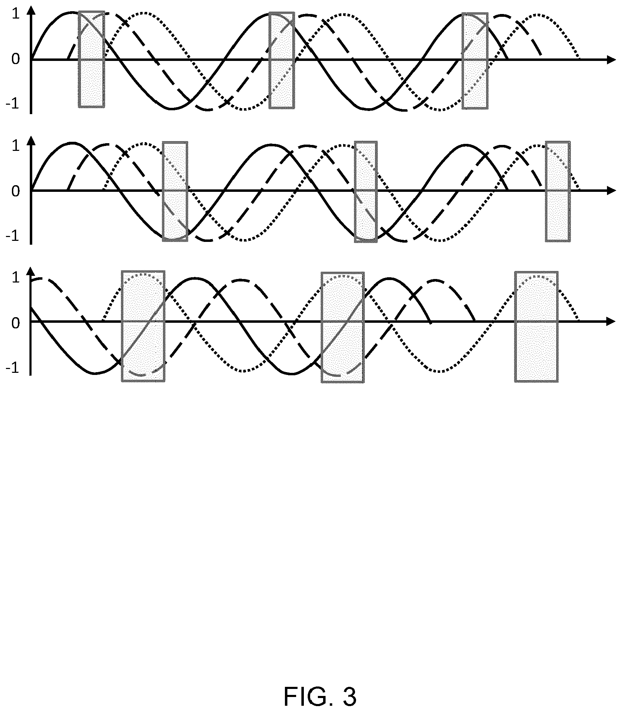

Fig. 3 also demonstrate the above concept of identification of time windows for radiotherapy treatment in which cell division rate of a tumor is at a peak, while cell division rate of healthy tissue is low. Specifically, there is shown curve representing the normalized circadian rhythm signals of the master clock (solid line), normal tissue (dashed line) and the tumor (dotted line).

The signals representing the different circadian rhythms of the body, organ and tumor are derived from different physiological signals, such as core body temperature, hormones or gene expression. However, the units and magnitudes of the signals are unimportant since it is the relative difference Δ between the tumor and normal tissue radiosensitivity that matters. In this case, it is assumed that the circadian rhythm signals represent the cell division rate or radiosensitivity.

In order to make the signal processing, simulations and interpretation easier, it helps to normalize the signals. The signals are normalized by dividing the signals with their amplitude and subtracting the average value. This way the normalized circadian rhythm signals vary between -1 and 1 in a periodic fashion, and hence, the difference varies between -1 < Δ < 1.

The shaded windows show ideal times during which the radiotherapy may be performed in an effective manner (i.e. when the relative radiosensitivity between the tumor and the normal tissue is high).

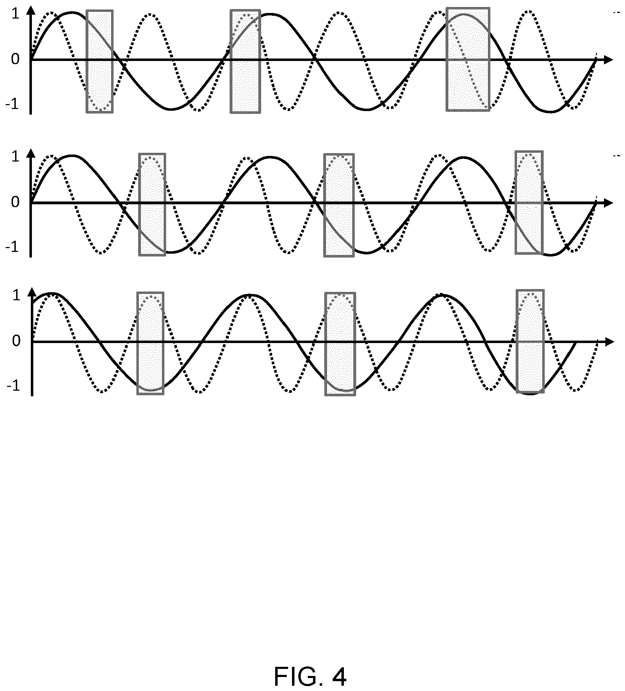

Of course, in the case of the tumor, the activity of the tumor may be dictated by an ultradian rhythm (as demonstrated in Fig. 4). As shown in Fig. 4, this means that the ultradian rhythm of the tumor (dotted line) may peak several times during one circadian rhythm period of the healthy tissue (solid line). This, of course, has implications for selection of treatment time windows.

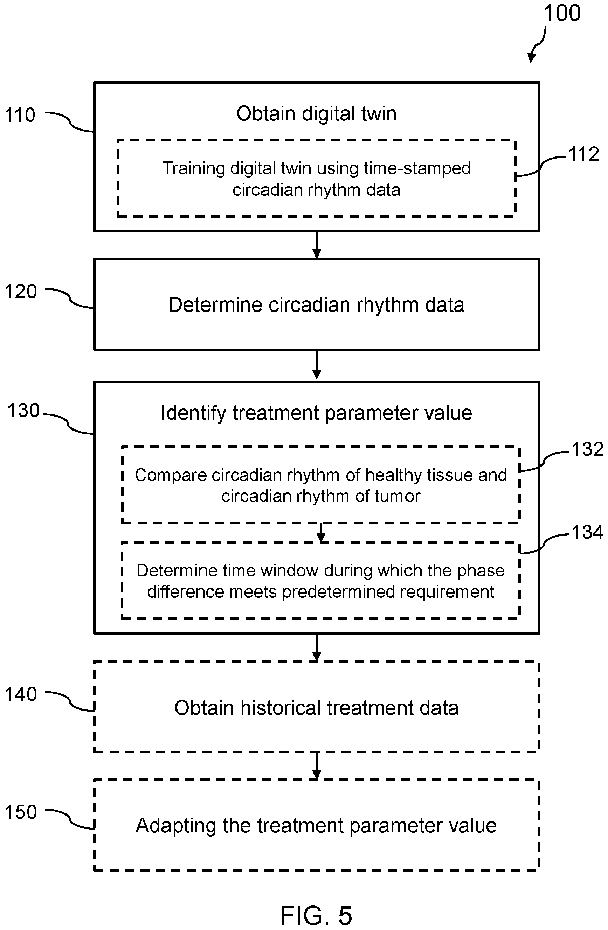

Moving on, Fig. 5 presents a flow diagram of a method 100 for controlling radiotherapy treatment of a subject. In other words, the method provides a means for controlling the execution of radiotherapy by generation of a treatment parameter value, or by control of one or more external parameters to treatment (i.e. factors that a subject or clinician may influence). The radiotherapy treatment of the subject may already be planned with a pre-defined set of parameter values, and the method is for augmentation/modification of said parameter values. Alternatively, the radiotherapy treatment may not yet be scheduled or planned, and the method generates a first set of parameter values (for review by a clinician).

In step 110, a digital twin is obtained. The digital twin is adapted to output predicted circadian rhythms of the subject. As stated above, a digital twin is a complex set of models that represent/reflect the state and future state of a physical twin (i.e. the subject). This enables the digital twin to be interrogated to understand interactions between internal processes, and external stimuli.

In this case, the digital twin is specifically adapted to output circadian rhythms (i.e. clocks) of the body. For example, the digital twin may determine characteristics/parameters of a body/peripheral clock of the body of the subject, both in the past, present and future, dependent on various physiological changes.

Accordingly, the digital twin enables an understanding of the subject, and specifically an interaction between changes to the subject and the circadian rhythm(s) of the subject.

Optionally, in sub-step 112 the digital twin adapted to represent circadian rhythms of the subject is trained using time-stamped circadian rhythm data of a tumor, time-stamped circadian rhythm data of healthy tissue, time-stamped circadian rhythm data of the body, and physiological data of the subject. The physiological data may comprise lifestyle data, environmental data, and therapeutic data of the subject.

Therefore, a fully trained subject-specific model is obtained that may be able to highlight links between physiological factors and circadian rhythms that may not be immediately apparent from inspection by a skilled person/clinician. This can enable accurate prediction and/or assessment of the circadian rhythms of the subject.

Alternatively, the digital twin may simply be obtained from a repository, where it was previously trained/generated. This may have occurred during previous treatment of the subject, or pre-emptively.

In step 120, circadian rhythm data of the subject is determined based on an output from the digital twin.

Indeed, as above, the digital twin is specifically adapted for outputting circadian rhythm data/information regarding the various circadian rhythms of the subject (i.e. a body clock, a peripheral clock, a clock of a tumor). In some cases, the digital twin may simply output the (predicted) circadian rhythm of the body of the subject, from which the circadian rhythms of specific organs can be derived (having a predictable lag from the main clock). In other cases, a full set of circadian rhythm data may be generated.

Additionally, circadian rhythm information may be gathered by direct testing of the subject. This may be performed by measuring the subject's body temperature, hormone levels, etc. Thus, such data can supplement the information supplied by the digital twin to determine an accurate set of circadian rhythm data.

In many embodiments, the circadian rhythm data of the subject may comprise circadian rhythm data of healthy tissue of the subject and circadian rhythm data of a tumor of the subject. As described above in relation to Figs 2-4, the difference between the circadian rhythm data of the healthy tissue and the tumor is a key factor in effectiveness of treatment. Thus, if this information is generated, treatment parameters may be appropriately selected. It should also be noted that the circadian rhythm of the tumor may also comprise ultradian rhythm data of the tumor (as is sometimes the case). That is, the rhythm of the tumor may have a period of less than 24 hours, and this may be accounted for by the digital twin.

In step 130, the circadian rhythm data is processed to identify a treatment parameter value for execution of radiotherapy treatment of the subject. Put another way, by analyzing the circadian rhythm data, an appropriate treatment parameter value can be determined that accounts for the circadian rhythms of the subject. As explained above, circadian rhythms of the subject have a large impact on the potential success of the treatment. Therefore, determining treatment parameter values (i.e. a time window of execution of the treatment, or a dosage regime) is informed by said circadian rhythm(s).

By way of specific example, if it is known that a subject's (main) circadian rhythm is at a trough during a time window, treatment execution may be scheduled during this time (i.e. treatment is scheduled when susceptibility of normal/healthy tissue to radiation is at a minimum, reducing the occurrence of associated issues). In a more complex example, a relative difference between circadian rhythm of healthy tissue and tumor tissue is determined, and a dosage regime (i.e. amount of radiation applied and time-intervals between application) can be selected so as to have a high dosage when a difference is larger than a threshold, and reduced when the difference is near a threshold.

Of course, processing the circadian rhythm data may comprise sub-step 132, in which the circadian rhythm data of the healthy tissue of the subject and of the tumor of the subject are compared. In this case, identifying the treatment parameter value is based on a result of the comparison.

This may be performed similarly to the methods described in relation to Figs 2-4. That is, the circadian rhythms may be normalized, and compared by taking a difference between the two. Alternatively, a weighted average of values representing the circadian rhythms may be performed.

In some embodiments, in sub-step 134, processing the circadian rhythm data comprises determining a time window (i.e. a period/multiple periods of time) during which the phase difference between the circadian rhythm of healthy tissue of the subject and the circadian rhythm of a tumor of the subject meets a predetermined requirement. Thus, the treatment parameter value is identified based on the identified time window.

The predetermined requirement may be a value set by a clinician, or may be when a normalized difference between the two rhythms exceeds a certain value (e.g. 0.5). In any case, the predetermined requirement may represent when the tumor is more susceptible to radiation than average, while the healthy tissue is less susceptible to radiation than average.

In some cases, the method may further comprise (optional) steps 140 and 150. In step 140, historical treatment data is obtained, which describes previous radiotherapy treatment of the subject. In step 150, the identified treatment parameter value is adapted based on the treatment data. The historical treatment data may comprise at least one of a treatment parameter value, a tumor response, a complication rate, and an immune system response.

In other words, the method may comprise additional steps that account for data associated with radiotherapy treatments performed in the past. This ensures that previous mistakes and/or successes in radiotherapy treatment are not disregarded.

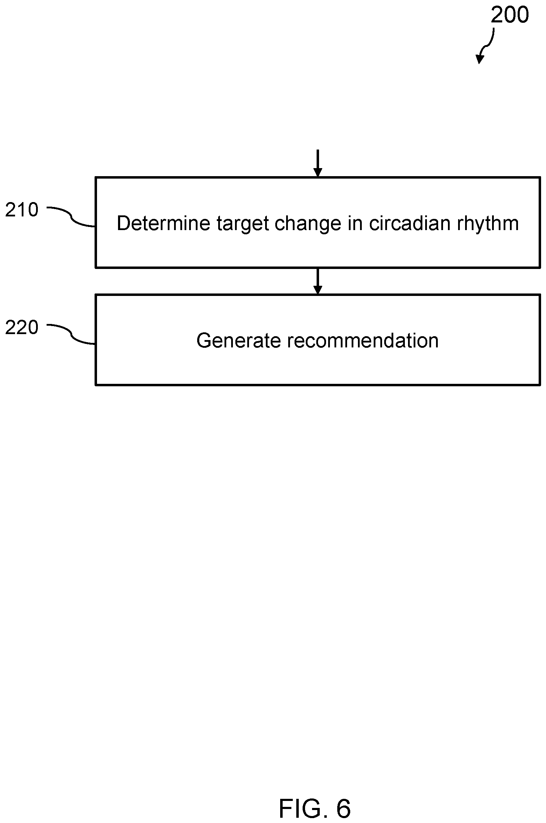

Fig. 6 presents a flow diagram of a method 200 according to an aspect of the invention for controlling radiotherapy treatment of a subject. The method 200 of Fig. 6 presents a way to control radiotherapy treatment that appreciates that subjects are not passive elements in the treatment process. That is, while treatment parameters may be altered, the subject may also be altered in order to improve chance of success of treatment within the bounds of identified treatment parameters.

In step 210, the circadian rhythm data is processed to determine a target change in the circadian rhythm of the subject. The change in the circadian rhythm of the subject may be a shift in the circadian rhythm (i.e. a movement in the peak of the circadian rhythm), a change in the impact of the circadian rhythm (i.e. a damping or increase in the amplitude of the circadian rhythm), or a disruption/suspension in the circadian rhythm.

Essentially, the target change may be adapted to increase an opportunity for successful treatment. This may be by manipulating the circadian rhythms so that a difference in cell division rates of healthy tissue and tumor tissue is increased at certain moments, or for ensuring a larger window during which the relative cell division rates are above a threshold value.

Specifically, the target change in the circadian rhythm may be adapted to increase a phase difference between a circadian rhythm of healthy tissue of the subject and a circadian rhythm of a tumor of the subject. This may be achieved by shifting the main circadian rhythm (i.e. body clock) of the subject so that a circadian rhythm of healthy tissue is shifted. Such a shift may have the aim of a peak of the circadian rhythm of the tumor being at the same time as the occurrence of a trough of the circadian rhythm of the healthy tissue.

In step 220, a recommendation is generated. The recommendation is based on the target change, the recommendation describing a change to an environment and/or a behavior of the subject. That is, if the recommendation is enacted (by the subject or a clinician) then the target change in the circadian rhythm may occur.

The recommendation may comprise at least one of a lighting regime, a temperature level regime, a nutrition regime, a hydration regime, a sleep regime, an exercise regime, and a medication regime. The skilled person would understand further factors that may impact the circadian rhythm of the subject in a predictable manner.

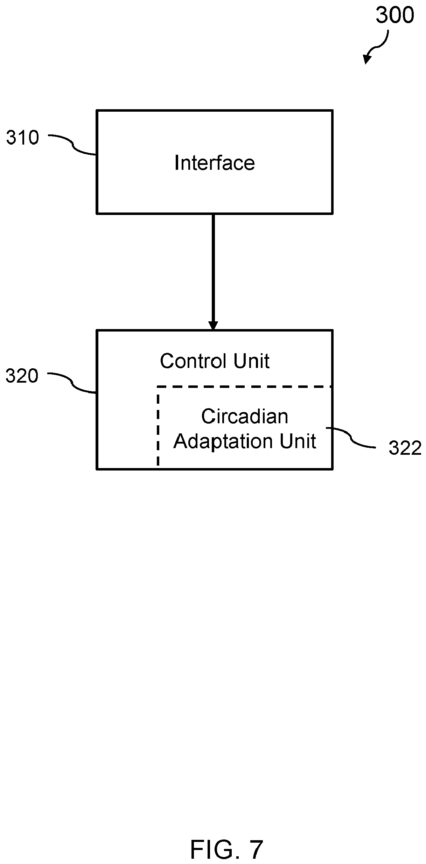

Fig. 7 presents a block diagram of a system for controlling radiotherapy treatment of a subject. The system comprises an interface 310 and a control unit 320, and may optionally further comprise a circadian adaptation unit 322. Each component may form part of the same device, or may be different connected devices.

The interface 310 is configured to obtain a digital twin adapted to output predicted circadian rhythms of the subject. The interface 310 may obtain the digital twin from a medical repository, a database, or a local memory. The digital twin may have been pre-generated/pre-trained, or may be trained by the interface 310 using time-stamped circadian rhythm and physiological data.

Subsequently, the control unit 320 receives an output from the digital twin via the interface 310, and is configured to determine circadian rhythm data of the subject based on the output from the digital twin. This may be achieved by deriving circadian rhythm data of various organs based on (main) circadian rhythm data of the subject. Alternatively, the digital twin may output circadian rhythm data of all relevant aspects of the subject.

The control unit 320 then processes the circadian rhythm data to identify a treatment parameter value for the execution of radiotherapy treatment of the subject. It is generally known how circadian rhythm impacts radiotherapy treatment (with certain tissue being more susceptible to radiotherapy at certain times of day). Therefore, the treatment parameter can be appropriately selected by understanding the circadian rhythm of the subject.

The (optional) circadian adaptation unit 322 is configured to process the circadian rhythm data to determine a target change in the circadian rhythm of the subject. The circadian adaptation unit 322 then generates a recommendation based on the target change, the recommendation describing a change to an environment and/or a behavior of the subject. That is, the circadian adaptation unit is configured to output (potentially to a display) a recommendation to prompt a change in an environment or behavior of the subject in the aim of altering the circadian rhythm of the subject to be more likely to receive successful treatment.

Overall, the system provides a means of controlling execution of treatment of the subject that accounts for the impact that circadian rhythm has on radiotherapy. Circadian rhythms are often complex, and changes are difficult to predict responsive to environmental and/or physiological changes. Thus, the digital twin enables an accurate prediction of the circadian rhythm for effective planning/selection of treatment parameter values.

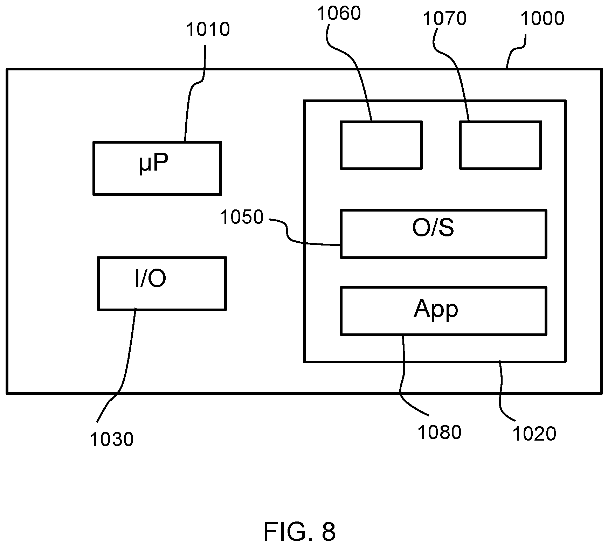

Moving on, Fig. 8 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method described in relation to Figs 5 and 6, and the system described in relation to Fig. 7, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for controlling radiotherapy treatment of a subject, the method comprising:
obtaining (110) a digital twin adapted to output predicted circadian rhythms of the subject;
determining (120) circadian rhythm data of the subject based on an output from the digital twin; and
processing the circadian rhythm data to identify (130) a treatment parameter value for execution of radiotherapy treatment of the subject.

2. The method of claim 1, further comprising:
processing (210) the circadian rhythm data to determine a target change in the circadian rhythm of the subject;
generating (220) a recommendation based on the target change, the recommendation describing a change to an environment and/or a behavior of the subject.

3. The method of claim 2, wherein the target change in the circadian rhythm is adapted to increase a phase difference between a circadian rhythm of healthy tissue of the subject and a circadian rhythm of a tumor of the subject.

4. The method of claim 2 or 3, wherein the recommendation comprises at least one of a lighting regime, a temperature level regime, a nutrition regime, a hydration regime, a sleep regime, an exercise regime, and a medication regime.

5. The method of any of claims 1-4, wherein the circadian rhythm data of the subject comprises circadian rhythm data of healthy tissue of the subject and circadian rhythm data of a tumor of the subject.

6. The method of claim 5, wherein processing the circadian rhythm data comprises comparing (132) the circadian rhythm data of the healthy tissue of the subject and of the tumor of the subject, and wherein identifying the treatment parameter value (130) is based on a result of the comparison.

7. The method of claim 6, wherein processing the circadian rhythm data comprises determining (134) a time window during which the phase difference between the circadian rhythm of healthy tissue of the subject and the circadian rhythm of a tumor of the subject meets a predetermined requirement, wherein identifying the treatment parameter value (130) is based on the identified time window.

8. The method of any of claims 1-7, further comprising:
obtaining (140) historical treatment data describing previous radiotherapy treatment of the subject; and
adapting (150) the identified treatment parameter value based on the treatment data.

9. The method of claim 8, wherein the historical treatment data comprises at least one of a treatment parameter value, a tumor response, a complication rate, and an immune system response.

10. The method of any of claims 1-9, wherein the treatment parameter value comprises one of a treatment execution window, and a dosage delivery regime.

11. The method of any of claims 1-10, further comprising training (112) the digital twin adapted to represent circadian rhythms of the subject using time-stamped circadian rhythm data of a tumor, time-stamped circadian rhythm data of healthy tissue, time-stamped circadian rhythm data of the body, and physiological data of the subject.

12. The method of claim 11, wherein the physiological data comprises lifestyle data, environmental data, and therapeutic data of the subject.

13. A computer program product comprising computer program code means which, when executed on a computing device having a processing system, cause the processing system to perform all of the steps of the method according to any of claims 1 to 12.

14. A system for controlling radiotherapy treatment of a subject, the system comprising:
an interface (310) configured to obtain a digital twin adapted to output predicted circadian rhythms of the subject;
a control unit (320) configured to determine circadian rhythm data of the subject based on an output from the digital twin, and process the circadian rhythm data to identify a treatment parameter value for the execution of radiotherapy treatment of the subject.

15. The system of claim 14, further comprising a circadian adaptation unit (322) configured to process the circadian rhythm data to determine a target change in the circadian rhythm of the subject, and generate a recommendation based on the target change, the recommendation describing a change to an environment and/or a behavior of the subject.
